# EUROPEAN PATENT APPLICATION

(11) **EP 1 774 931 A2**
(43) Date of publication of application: **18.04.2007**
(21) Application number: 06021317.0
(22) Date of filing: 11.10.2006
(51) Int. Cl.: A61F 5/445, A61F 2/00

(54) **Controlled evacuation ostomy appliance**

(30) Priority: 11.10.2005 US 725504 P
(71) Applicant: Bristol-Myers Squibb Company, New York, N.Y. 10154 (US)
(72) Inventor: Davies, Geraint, Cambridge CB2 2QS (GB); Cline, John, New Brunswick, New Jersey 08901 (US); Gregory, Christopher C., Newtown, Pennsylvania 18940 (US); Cucknell, Alan, Cambridge CB1 3SL (GB); Scarfe, Julian, Cambridge CB2 2HH (GB); Cauwood, Pete, Waterbeach, Cambridge CB5 9HP (GB)
(74) Representative: Holmes, Miles Keeton

(57) **Abstract**

A controlled evacuation ostomy appliance (10) includes an attachment device (16) for attaching the appliance to a wearer, a seal (12) for sealing against stomal tissue (14), and a restrainer (18). The restrainer restrains the seal relative to the attachment device and transfers a sealing force (or its reaction) between the attachment device and the seal.

## Description

### FIELD OF THE INVENTION

The present invention relates to the field of ostomy appliances, and in particular to such appliances which can be used to control stomal discharge, occasionally referred to as controlled evacuation appliances.

### BACKGROUND TO THE INVENTION

The creation of an ostomy (stoma) is the therapy for many sufferers of diseases or injury of the gastrointestinal or urinary tract. An ostomy is the rerouting of the tract through the abdominal wall to outside the patient's body. Once a stoma has been created, the patient must, frequently for the rest of his or her life, use a device worn on the body for capturing or containing the body waste. This has traditionally been done with a bag or pouch attached to the body with adhesive patches or constricting belts. However, the wearing of such a pouch can be an extremely embarrassing experience for many ostomates. Also a pouch may require significant changes to a person's public and personal activities.

A controlled evacuation appliance offers the potential for an ostomate to return to some form of normally. The appliance is used to block the stoma mouth, in order to store the liquid and/or solid stool temporarily inside the tract. The appliance is deactivatable and/or removable manually when the ostomate desires to discharge the stool from the stoma. A design feature which distinguishes a controlled evacuation appliance from a conventional ostomy pouch is the presence of a stoma seal, for blocking the stoma mouth. However, there are many practical and challenging difficulties associated with implementing a cost efficient, yet effective, comfortable and unobtrusive stoma seal. It is believed that this may be one of the reasons why controlled evacuation appliances have hitherto not found widespread use.

Reference may be made to the controlled evacuation devices described in U.S. Patent No. 6,723,079 and U.S. Publication No. 2004/0193122. The devices generally comprise a flexible stoma seal contained in a rigid, closed, U-shaped caplike structure for supporting the seal.

### SUMMARY OF THE INVENTION

Broadly speaking, the present invention relates to a restraining structure for a stoma seal in a controlled evacuation ostomy appliance. The restraining structure serves to (i) restrain the stoma seal with respect to a body attachment device, and/or (ii) transfer a sealing force (or its reaction force) between the body attachment device and the stoma seal.

In one form, at least a portion of the restraining structure is flexible (e.g., non-rigid). The flexible portion acts in tension to generate, apply or transfer the sealing force (or its reaction) to the stoma seal.

in another form, the restraining structure only partly covers the stoma seal. The structure includes one or more apertures that expose the uncovered parts. The structure may be substantially entirely flexible, or partly flexible, or substantially entirely non-flexible.

In another form, the restraining structure has a dome-shape, at least in use. The term "dome" is used herein to mean any shape that has a smoothly curved profile, whether the curve is uniform or varying. The structure may be substantially entirely flexible, or partly flexible, or substantially entirely non-flexible.

Although the above features have been defined independently, any two or more of the above features may be used in combination. The present invention explicitly envisages all such possible combinations.

Additional or alternative aspects, features and advantages of the invention may be defined, or apparent from, the following description, claims and drawings.

### BRIEF DESCRIPTION OF THE DRAWINGS

Fig. 1 is a schematic cross section showing a first example of a controlled evacuation ostomy appliance in use on the body.
Fig. 2 is a schematic cross section showing a second example of a controlled evacuation ostomy appliance in isolation.
Fig. 3 is a schematic cross section showing a third example of a controlled evacuation ostomy appliance in isolation.
Fig. 4 is a schematic rear view showing a fourth example of a controlled evacuation ostomy appliance in isolation.
Fig. 5 is a schematic cross section showing a fifth example of a controlled evacuation ostomy appliance in isolation.
Fig. 6 is a schematic cross section showing a sixth example of a controlled evacuation ostomy appliance in isolation.

### DETAILED DESCRIPTION OF THE INVENTION

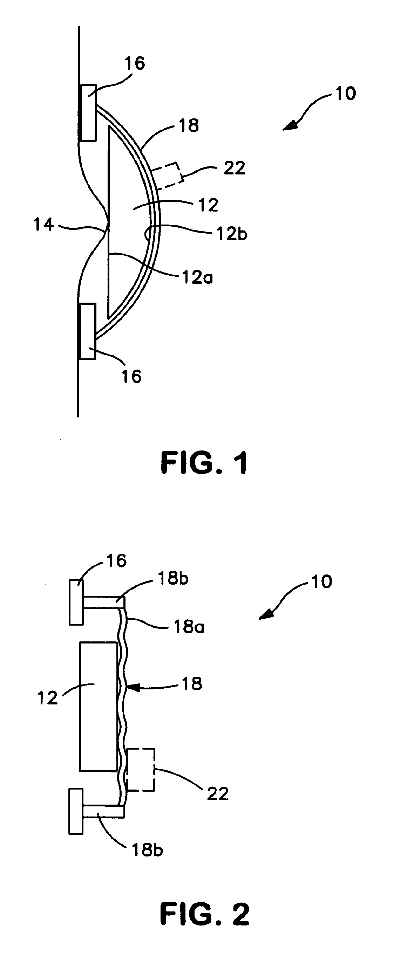

Referring to Fig. 1, a controlled evacuation ostomy appliance 10 is illustrated in accordance with the principles of the present invention. The appliance comprises a stoma seal (depicted schematically at 12) for blocking or occluding the mouth of a wearer's stoma 14, in order to substantially contain faecal matter internally n the intestinal tract.

The stoma seal 12 may at least partly enter the stoma 12, or the seal may be substantially non-entrant. A non-entrant seal contacts substantially only externally facing stomal tissue. The stoma seal 12 comprises a first face (or portion) 12a that faces generally towards the stoma 14, and a second face (or portion) 12b that faces generally away from the stoma 14.

The stoma seal 12 may be inflatable, or non-inflatable. The stoma seal 12 may be compressible or non-compressible, The stoma seal 12 may, by way of example, comprise a membrane, or a plurality of membranes, or a foam. The stoma seal 12 may be gas-impermeable or gas-permeable. The stoma seal 12 is substantially impermeable to liquid and solid fecal matter.

The appliance 10 further comprises an attachment device 16 for attachment to the body. The attachment device comprises a wafer or pad of a skin-friendly adhesive. The attachment device 16 is permanently attached to the appliance 10 to form a so-called "one-piece" appliance, or at least a part of the attachment device 16 is releasably attached (or attachable) to the appliance 10 to form a so-called "two-piece" appliance. Alternatively to an adhesive attachment device 16, the attachment device 16 may comprise a belt-attachment including a belt to be worn by the wearer, an item of clothing incorporating the appliance 10, or mechanical or magnetic attachment devices that form an attachment to permanent features of the wearer.

The appliance 10 further comprises a restrainer (restraining structure) 18 for supporting the stoma seal 12. The restrainer 18 may itself be supported directly or indirectly by the attachment device 16, for example at the periphery of the restrainer 18. At least a portion of the restrainer 18 may be flexible, e.g., substantially non-rigid. The flexible portion may be elastic, or non-elastic (e.g., non extendable). The restrainer 18 functions to support the stoma seal 12 by its second face (portion) 12b The restrainer 18 acts at least partly under tension, to transfer a sealing force (or its reaction force) directly or indirectly between the stoma seal 12 and the attachment device 16. At least the flexible portion of the restrainer 18 acts under tension. The reaction to the sealing force is borne by the attachment device 16 mounting the appliance 10 with respect to the wearer's body.

Depending on the design of the appliance, the flexibility of (at least a portion 18a of) the restrainer 18 would enable the appliance 10 to be especially unobtrusive when worn under clothing. The flexibility enables hard edges of a rigid cap-like structure to be avoided, as such hard edges could show through a wearer's clothing. The flexibility may also reduce the degree of rigidity of the appliance 10 as a whole. Reducing the rigidity makes the appliance 10 less obtrusive, and also allows the appliance to conform more easily to the shape of the wearer's body. Reducing the rigidity would also enable the appliance to be collapsible to some extent when not in use, and thus reduce the size of the appliance 10 for storage, transportation and packaging.

Depending on the design of the appliance, the use of tension in the flexible (portion of the) the restrainer 18 may enable the restrainer to have a low profile while still providing an effective structure for transferring a sealing force (or its react on) directly or indirectly between the attachment device 16 and the stoma seal 12.

The restrainer 18 is adjustable by the wearer, in order to adjust the sea ing force applied by the stoma seal 12 against the stoma 14, For example, an adjustable tensioner (depicted schematically at 22) is included for adjusting the tension in the flexible portion of the restrainer 18. Alternatively, the sealing force may be predetermined by the relative dimensions of the appliance 10.

The restrainer 18 may be configured to apply a uniform force over the area of the seal 12, or the restrainer may be configured to apply a non-uniform force. For example, the tension in the flexible portion of the restrainer 18 may vary at different points.

The restrainer 18 (or its flexible portion) may have a closed loop configuration, or it may have a non-closed loop configuration.

Referring to Fig. 1, the restrainer 18 is entirely flexible. For example, the restrainer 18 consists of a flexible sheet, or a web or net, that is attached to the attachment device 16 at the periphery of the restrainer 18. The restrainer 18 acts as a flexible sling or flexible cup to restrain the stoma seal 12 with respect to the attachment device 16.

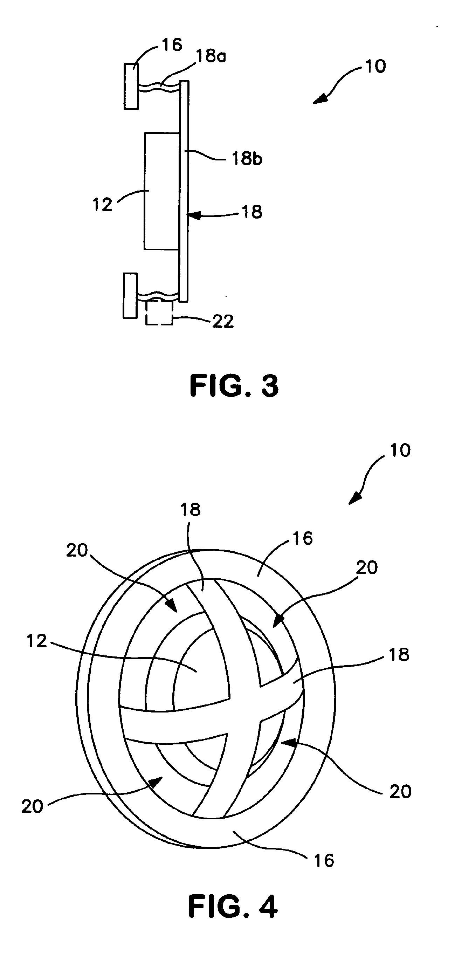

Alternatively, referring to Figs. 2 and 3, only a portion 18a of the restrainer is flexible while another portion 18b of the restrainer is substantially non-flexible (e.g., rigid). In Fig. 2, the non-flexible portion 18b comprises a peripheral (e.g., cylindrical) wall carried by the attachment device 16. The flexible portion 18a comprises a flexible rear portion supported by the non-flexible portion. The flexible portion 18a supports the stoma seal 12. In Fig. 3, the non-flexible portion 18b comprises a rear portion that supports the stoma seal 12. The flexible portion 18a comprises a flexible and/or collapsible intermediate portion between the non-flexible portion 18b an d the attachment device 16. The flexible intermediate portion provides a degree of shape independence between the attachment portion and the non-flexible rear portion 18b, such that the relatively rigid rear portion 18b would not restrict the conformability of the attachment device 16 to conform to the shape of the wearer's body.

The restrainer 18 may cover the stoma seal 12 substantially entirely, or the restrainer 18 may only partly cover the stoma seal 12. For example, referring to Fig. 4, the restrainer 18 could be frame-like, or cage-like, and comprise one or more apertures 20. The apertures expose the stoma seal 12. The restrainer 18 may be substantially entirely flexible, or partly flexible or non-flexible (e.g., rigid). For example, a flexible restrainer 18 may be formed by one or more straps extending over the stoma seal 12.

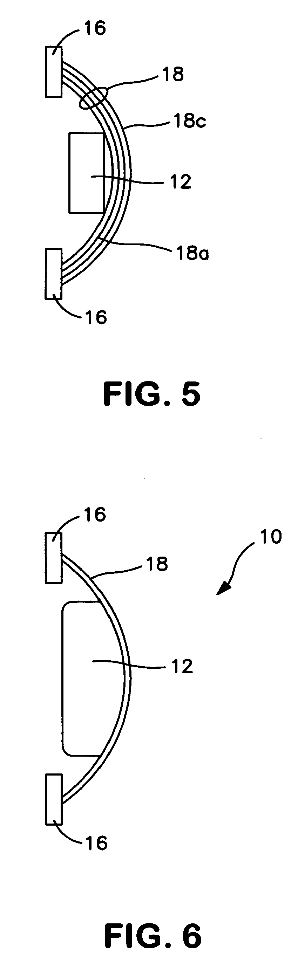

Fig. 5 illustrates a hybrid embodiment combining the examples of Figs. 1 and 4. The restrainer 18 comprises a restrainer frame 18c (similar to Fig. 4) which supports a restrainer sheet 18a (similar to Fig. 1). The restrainer sheet 18a may be more flexible than the restrainer frame 18c.

In a further modification of Fig. 4, exposed portions of the stoma seal 12 may be protected by a flexible cover (not shown) that is positioned between the stoma seal 12 and the restrainer 18, or outside the restrainer 18. Such a cover does not provide a restraining function.

Referring to Fig. 6, the restrainer 18 comprises a dome-like structure. The restrainer 18 is entirely flexible (similar to Fig. 1), or partly flexible or non-flexib e (e.g., rigid). The use of a dome-like shape enables the appliance to have a "soft" profile by avoiding sharp corners or edges. Such a profile enables the appliance to be relatively unobtrusive when worn under clothing.

The foregoing description is merely illustrative of preferred forms of the invention. Many modifications, improvements and equivalents may be used within the scope and/or spirit of the invention.

## Claims

1. A controlled evacuation ostomy appliance comprising:
an attachment device for attaching the appliance to the wearer's body;
a seal for sealing against a wearer's stoma to obstruct discharge of body waste from the stoma; and
a restraining structure for restraining the seal with respect to the attachment device and for transferring a sealing force or a sealing force reaction between the attachment device and the seal, the restraining device comprising a flexible portion.

2. The appliance according to claim 1, wherein, in use, the flexible portion of the restraining structure acts in tension in order to transfer said sealing force or sealing force reaction.

3. The appliance according to claim 1 or 2, wherein the restraining structure is substantially entirely flexible.

4. The appliance according to claim 1 or 2, wherein the restraining structure further comprises a substantially non-flexible portion.

5. The appliance according to claim 4, wherein the non-flexible portion comprises a peripheral wall, and the flexible portion comprises a rear portion supported by the peripheral wall.

6. The appliance according to claim 4, wherein the non-flexible portion comprises a rear wall portion, and the flexible portion comprises a flexible intermediate portion between the rear wall portion and the attachment device.

7. The appliance according to any preceding claim, wherein the flexible portion is substantially inelastic.

8. The appliance according to any preceding claim, wherein the restraining structure substantially covers the seal.

9. The appliance according to any of claims 1 to 7, wherein the restraining structure partly covers the seal.

10. The appliance according to any preceding claim, wherein the restraining structure, at least when in use, has a dome shape.

11. A controlled evacuation ostomy appliance, comprising:
an attachment device for attaching the appliance to the wearer's body
a seal for sealing against a wearer's stoma to obstruct discharge of body waste from the stoma; and
a restraining structure for restraining the seal with respect to the attachment device and for transferring a sealing force or a sealing force reaction between the attachment device and the seal, wherein the restraining structure partly covers the seal.

12. The appliance according to claim 11, wherein the restraining structure comprises a flexible portion.

13. The appliance according to claim 12, wherein the flexible portion comprises at least one strap or mesh, and defines an open structure.

14. The appliance according to any of claims 11 to 13, wherein the restraining structure is cage-like or frame-like.

15. The appliance according to any of claims 11 to 14, wherein the restraining structure comprises one or more apertures.

16. A controlled evacuation ostomy appliance comprising:
an attachment device for attaching the appliance to the wearer's body;
a seal for sealing against a wearer's stoma to obstruct discharge of body waste from the stoma; and
a restraining structure for restraining the seal with respect to the attachment device and for transferring a sealing force or a sealing force reaction between the attachment device and the seal, wherein the restraining structure has a dome-shape at least in use.

17. The appliance according to claim 16, wherein the restraining structure comprises a flexible portion.

18. The appliance according to claim 16 or 17, wherein the restraining structure defining the dome-shape is substantially rigid.
